# EUROPÄISCHE PATENTSCHRIFT

(11) **EP 0 385 925 B1**
(45) Veröffentlichungstag und Bekanntmachung des Hinweises auf die Patenterteilung: **26.05.1993**
(21) Anmeldenummer: 90810084.5
(22) Anmeldetag: 07.02.1990
(51) Int. Cl.: A61F 2/06, A61L 27/00, D04H 1/54

(54) **Geflochtene Gefäss-Prothese**
Plaited vascular prosthesis
Prothèse vasculaire tressée

(30) Priorität: 28.02.1989 CH 740/89
(43) Veröffentlichungstag der Anmeldung: 05.09.1990
(73) Patentinhaber: GEBRÜDER SULZER AKTIENGESELLSCHAFT, CH-8401 Winterthur (CH); IMS-BIOPUR AG, CH-8807 Freienbach (CH)
(72) Erfinder: Bittmann, Peter, Dr., CH-8057 Zürich (CH); Dittes, Peter, CH-8635 Oberdürnten (CH); Flückiger, Hans, CH-8618 Oetwil am See (CH)

(56) Entgegenhaltungen:
- EP-A- 0 248 246
- EP-A- 0 248 247
- WO-A-80/02641
- WO-A-82/01647
- FR-A- 2 416 686
- GB-A- 1 183 497
- GB-A- 2 115 776
- GB-A- 2 139 897
- US-A- 4 191 218

## Beschreibung

Die Erfindung betrifft eine geflochtene Gefäss-Prothese, deren Flechtwerk aus multifilen Fäden eines langzeitstabilen Kunststoffes besteht.

Nach neueren Untersuchungen besteht eine der Hauptforderungen an Gefässprothesen - insbesondere an solche mit kleinen Durchmessern, beispielsweise von 7 mm und kleiner - darin, dass ihre radiale Dehnbarkeit an diejenige eines natürlichen Gefässes möglichst weitgehend angepasst sein soll. Die Wände künstlicher Gefässprothesen sollen daher elastisch sein, insbesondere in radialer Richtung.

Um die Gefahr der Thrombenbildung zu vermindern oder zu vermeiden, werden darüberhinaus die Prothesenwände entweder mit lebenden, patienteneigenen Endothel-Zellen oberflächlich beschichtet (US-PS 4,804,381 und 4,804,382) oder beimpft (seeding-Verfahren; James C. Stanley, Linda M. Graham & William E. Burkel: "Endothelial Cell Seeding of Synthetic Vascular Prostheses" in "Modern Vascular Grafts", McGraw-Hill, 1987). Das Seeding-Verfahren kann auch mit einem aus den Mikrogefässen des Fettgewebes des Patienten gewonnenen Zellengemisch durchgeführt werden (Steven P. Schmidt et al: "Microvascular Endothelial Cell Seeding of Small-Diameter Dacron ^{TM} Vascular Grafts", Journal of Investigative Surgery, Volume 1, 1988, pp. 35-44).

Sowohl das Verfahren der oberflächlichen Beschichtung ("Lining") als auch das Verfahren der Beimpfung ("Seeding") sind bei Prothesen angewandt worden, die als Schläuche gefertigt worden sind und entweder aus expandiertem Polytetrafluorethylen (e-PTFE) oder aus textiler Web- oder Wirkware, bestehen; diese Prothesen sind in allen Fällen nicht radial dehnbar.

Darüberhinaus sind in letzter Zeit Prothesen aus elastischen Kunststoffen entwickelt worden, insbesondere aus mikroporösen Polyurethanen (PUR). Neuere Veröffentlichungen haben gezeigt, dass jedoch elastische Kunststoffe im Körper nicht langzeitstabil sind (Michael Szycher, Ph.D. "Biostability of Polyurethane Elastomers: A Critical Review", Journal of Biomaterials Applications, Volume 3 - October 1988).

Als langzeitstabile Kunststoffe haben sich im Laufe der Jahrzehnten u.a. thermoplastische Kunststoffe, wie z.B. Polyester (PES) - von diesen insbesondere Polyethylenterephtalat (PETP) - Polyethylen (PE) und Polypropylen (PP) erwiesen. Diese Kunststoffe sind jedoch praktisch unelastisch, so dass Schlauchprothesen, die bisher aus diesen Materialien gefertigt werden, keine radiale Dehnbarkeit in dem erforderlichen Masse aufweisen.

Eine Prothese der eingangs genannten Art ist bekannt aus der US-PS 4,441,215 (= wo 82/01647). Bei der dortigen Konstruktion wird der geflochtene Schlauch aussen mit einem Rohr aus einem elastischen Material umgeben, um den Schlauch gegen ein Zusammendrücken von aussen zu schützen und ihn darüberhinaus "abzudichten". Eine radiale Dehnbarkeit ist bei dieser Prothese nicht vorhanden.

Andere bekannte Prothesen aus textiler Maschenware sind ausserordnetlich dicht gewoben oder kettengewirkt. Dadurch sind sie ebenfalls nicht in genügendem Masse radial dehnbar.

Weiterhin ist von einer Prothese aus textiler Maschenware Widerstandsfähigkeit gegen ein Ausreissen beim Nähen, die sogenannte Nahtausreisskraft, zu fordern; diese soll z.B. nach bisherigen Untersuchungen mindestens 10 Newton (N) betragen, wenn der Einstich der Nadel in einem Abstand von 1 mm von der Schnittkante erfolgt.

Aufgabe der Erfindung ist es, eine durch Beimpfung mit Zellen zu beladende langzeitstabile Gefässprothese, insbesondere für kleinlumige Gefässe zu schaffen, die eine radiale Dehnbarkeit hat, die soweit wie möglich an diejenige eines natürlichen Gefässes angenähert ist; darüberhinaus soll die neue Prothese die Anforderung der vorstehend definierten Mindest-Nahtausreisskraft erfüllen.

Mit der Erfindung wird diese doppelte Aufgabe dadurch gelöst, dass die Fäden des Flechtwerks texturiert sind, und dass ferner mindestens in einem Teil der Kreuzungspunkte des Geflechtes mindestens einzelne der Filamente der sich kreuzenden Fäden fest miteinander verbunden sind.

Durch die Texturierung erhalten die Filamente bzw. Fäden des Geflechtes, das relativ locker und lose ist, um die Zellen der Beimpfung "aufzunehmen", die erforderliche Elastizität, die dem Prothesenschlauch die notwendige radiale Dehnbarkeit verleiht. Die feste Verbindung der sich kreuzenden Fäden mindestens an einer Anzahl Kreuzungsstellen gewährleistet die geforderte Nahtausreisskraft.

Als bevorzugte Materialien für das Geflecht aus texturierten Fäden haben sich Fasern aus texturierbaren, thermoplastischen Kunststoffen mit einem Schmelzbereich, insbesondere Polyehtylenterephtalat oder Polyethylen, bewährt; der Durchmesser eines einzelnen Filamentes kann zwischen 10 und 100 µm, vorwiegend zwischen 10 und 20µm, betragen.

Als feste Verbindung der Filamente bzw. Fäden hat sich ein Verkleben mit einem vorzugsweise elastischen Klebstoff, z.B. Polyurethan, als geeignet erwiesen. Es ist jedoch auch ein "Verschweissen" möglich, bei dem das Geflecht aus Fäden unterschiedlicher Materialien besteht, von denen mindestens eines einen tiefen Schmelzbereich hat; das Verschweissen der Filamente erfolgt dabei dadurch, dass die Fäden aus Materialien mit den tieferen Schmelzbereichen bei einer Wärmebehandlung zumindestens angeschmolzen werden und anschliessend wieder erstarren.

Selbstverständlich müssen alle Materialien der neuen Prothese biokompatibel sein; hierfür ist besonders ein Geflecht aus Polyehtylenterephtalat-Filamenten geeignet. Als zu schmelzendes Material bei der Herstellung der "Schweiss"-Verbindung hat sich Polyethylen bewährt. Es ist aber auch möglich, dass Geflecht aus Polyester-Fäden mit unterschiedlichen Schmelzbereichen herzustellen, die ein Gemisch mit einem eutektischen Schmelzbereich bilden oder bei denen tiefer schmelzende Komponenten als Schweissmaterial wirken.

Im folgenden wird die Erfindung anhand eines Ausführungsbeispiels näher erläutert.

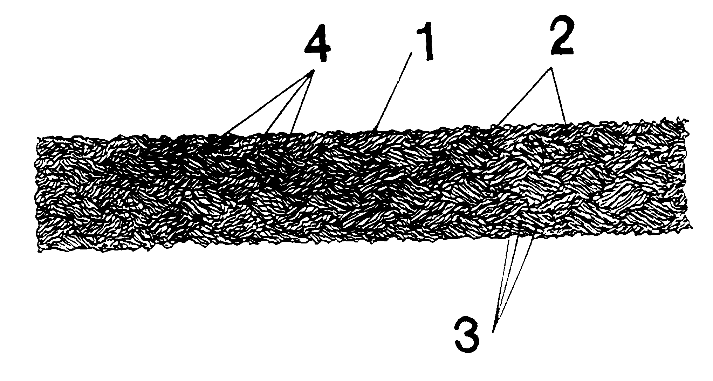

Die einzige Figur zeigt schematisch ein Stück einer Gefässprothese in einer Vergrösserung.

Das Flechtwerk der Prothese 1 besteht aus Fäden 2, die ihererseits aus Filamenten oder Fibrillen 3 gebildet werden. Mindestens bei einer Anzahl der Kreuzungspunkte 4 sind einzelne der Fibrillen der sich kreuzenden Fäden 2 fest miteinander verbunden, z.B. miteinander verklebt, was aus der Darstellung nicht zu entnehmen ist. Zwischen den Kreuzungspunkten 4 sind bei dem relativ lockeren Geflecht "freie Flächen" eines gitterartigen Netzes vorhanden, in dem die Zellen nach dem Beimpfen mit Hilfe des Seeding-Verfahrens haften.

In dem gezeigten Beispiel beträgt der Flechtwinkel gegenüber der Längsachse der Prothese etwa 30°; er kann jedoch auch grössere Werte bis beispielsweise 60° annehmen.

Die gezeigte Prothese besteht aus Polyethylenterephtalat-Fäden, die sich aus einzelnen Filamenten mit einem Durchmesser von etwa 20 µm zusammensetzen; das Geflecht ist durch Kleben fixiert, wobei als Klebstoff ein elastisches Polyurethan dient.

Die Herstellung der neuen Prothese kann beispielsweise auf folgende Weise erfolgen:

32 Fäden des genannten Kunststoffes mit einem Titer von 78 decitex, die je aus 24 Fibrillen oder Filamenten mit dem genannten Durchmesser bestehen und hochelastich texturiert worden sind, werden auf einen glatten zylindrischen Stab von 8 mm Durchmesser geflochten. Ist die gewünschte Länge des Flechtwerkes erreicht, so wird das Geflecht vom Stab abgezogen und auf einen zweiten Stab mit einem geringeren Durchmesser von beispielsweise 5 mm aufgezogen. Durch kurzzeitiges Tauchen in kochendes, bidestilliertes Wassen wird das Geflecht auf diesem zweiten Stab geschrumpft, um seine Texturierung zu stabilisieren.

Anschliessend wird das Geflecht nunmehr in einer 5%-igen wässrigen Lösung eines zellfreundlichen, im Handel erhältlichen Waschmittels längere Zeit, beispielsweise während 24 Stunden, gewaschen und anschliessend in mehrmals erneuertem Wasser gespült, wobei das letzte Spülmittel aus bidestilliertem Wasser besteht; es erfolgt nun eine Trocknung bei etwa 150° C.

Um die Benetzbarkeit der Fibrillen zu erhöhen, wird das Geflecht in einem aus der Färbetechnik von Kunststoff-Fasern bekannten Färbe-Beschleuniger (Carrier) zum Quellen gebracht. Ein derartiger Beschleuniger ist ein organisches Lösungsmittel, beispielsweise Dichlor-Methan oder auch Tetrachlorethylen, Dichlorbenzol oder Trichlorbenzol.

Das gequollene Geflecht wird anschliessend zum Verkleben seiner Kreuzungspunkte in eine 5%-ige Polyurethan-(PUR)-Lösung in Dimethylformamid getaucht und nach Durchtränkung mit der Lösung erfolgt ein Trocknen mit strömender Luft in einer Laminar-flow-Kabine.

Um Lösungsmittelreste zu entfernen unterwirft man die Prothese abschliessend bei 50° C einem Vakuum von etwa 1 mm Hg, wobei sie für diese Evakuierung zuvor in eine dampfdurchlässige Folie eingepackt worden ist.

Das fertige Produkt ist eine elastische Gefässprothese von etwa 5 mm Durchmesser mit einer Compliance - das ist ein Mass für die radiale Dehnbarkeit und wird gemessen als Quotient aus der relativen Durchmesser-Veränderung Δ D/D geteilt durch den Druckunterschied Δp - von etwa 4.10 ⁻⁶ pro Pa Druckunterschied.

## Patentansprüche

1. Geflochtene Gefäss-Prothese, deren Flechtwerk aus multifilen Fäden eines langzeitstabilen Kunststoffes besteht, dadurch gekennzeichnet, dass die Fäden (2) des Flechtwerkes texturiert sind,und dass ferner mindestens in einem Teil der Kreuzungspunkte (4) des Geflechtes mindestens einzelne der Filamente (3) der sich kreuzenden Fäden fest miteinander verbunden sind.

2. Gefäss-Prothese nach Anspruch 1, dadurch gekennzeichnet, dass die feste Verbindung durch Verkleben mit Hilfe eines Klebstoffes hergestellt ist.

3. Gefäss-Prothese nach Anspruch 2, dadurch gekennzeichnet, dass der Klebstoff aus elastischem Material besteht.

4. Gefäss-Prothese nach Anspruch 1, dadurch gekennzeichnet, dass das Geflecht aus Fäden (2) unterschiedlicher Materialien besteht, von denen mindestens eines einen tiefen Schmelzbereich hat.

5. Gefäss-Prothese nach einem der Ansprüche 1 bis 4, dadurch gekennzeichnet, dass das Geflecht aus Fäden (2) von Polyethylenterephtalat-Filamenten besteht.

6. Gefäss-Prothese nach Anspruch 4 und 5, dadurch gekennzeichnet, dass das Material mit tiefem Schmelzbereich Polyethylen ist.

## Claims

1. A braided vascular prosthesis, in which the braiding is made of multifilar threads of a plastics having long-term stability, characterised in that the threads (2) of the braiding are textured and at least some of the filaments (3) of the intersecting threads are permanently connected at at least some of the points of intersection (4) of the braiding.

2. A vascular prosthesis according to claim 1, characterised in that the permanent connection is made by sticking with an adhesive.

3. A vascular prosthesis according to claim 2, characterised in that the adhesive consists of elastic material.

4. A vascular prosthesis according to claim 1, characterised in that the braiding is made of threads (2) of various materials, at least one of which has a low melting range.

5. A vascular prosthesis according to any of claims 1 to 4, characterised in that the braiding is of threads (2) of polyethylene terephthalate filaments.

6. A vascular prosthesis according to claim 4 or 5, characterised in that the material having a low melting range is polyethylene.

## Revendications

1. Prothèse vasculaire tressée, dont le treillis est composé de fils multifils en une matière plastique stable à long terme, caractérisée en ce que les fils (2) du treillis sont texturés, et que de plus dans une partie au moins des points d'entrecroisement (4) du treillis, quelques filaments individuels (3) au moins des fils qui s'entrecroisent sont solidement reliés les uns aux autres.

2. Prothèse vasculaire suivant la revendication 1, caractérisée en ce que la jonction solide est réalisée par collage à l'aide d'une colle.

3. Prothèse vasculaire suivant la revendication 2, caractérisée en ce que la colle consiste en une matière élastique.

4. Prothèse vasculaire suivant la revendication 1, caractérisée en ce que le treillis est constitué de fils (2) en matières différentes, dont au moins une possède une basse plage de fusion.

5. Prothèse vasculaire suivant l'une des revendications 1 à 4, caractérisée en ce que le treillis est constitué de fils (2) en filaments de polyéthylène-téréphtalate.

6. Prothèse vasculaire suivant les revendications 4 et 5, caractérisée en ce que la matière ayant une basse plage de fusion est un polyéthylène.
